# EUROPÄISCHE PATENTSCHRIFT

(11) **EP 3 481 805 B1**
(45) Veröffentlichungstag und Bekanntmachung des Hinweises auf die Patenterteilung: **18.01.2023**
(21) Anmeldenummer: 17736603.6
(22) Anmeldetag: 28.06.2017
(51) Int. Cl.: C07D 213/803, C07D 213/79, C07D 213/61, C07F 3/06

(54) **VERFAHREN ZUR HERSTELLUNG HALOGENIERTER PYRIDINDERIVATE**
METHOD FOR THE PREPARATION OF HALOGENATED PYRIDINE DERIVATIVES
PROCEDE DE FABRICATION DE DERIVES DE PYRIDINE HALOGENEE

(30) Priorität: 05.07.2016 EP 16178022
(43) Veröffentlichungstag der Anmeldung: 15.05.2019
(73) Patentinhaber: Bayer CropScience Aktiengesellschaft, 40789 Monheim am Rhein (DE)
(72) Erfinder: MOSRIN, Marc, 50935 Köln (DE); FISCHER, Rüdiger, 50259 Pulheim (DE); HAGER, Dominik, 40789 Monheim (DE); HOFFMEISTER, Laura, 40593 Düsseldorf (DE); KAUSCH-BUSIES, Nina, 51467 Bergisch Gladbach (DE); WILCKE, David, 40235 Düsseldorf (DE); WILLOT, Matthieu, 40215 Düsseldorf (DE)
(74) Vertreter: BIP Patents
(86) Internationale Anmeldenummer: PCT/EP2017/065989
(87) Internationale Veröffentlichungsnummer: WO 2018/007224

(56) Entgegenhaltungen:
- WO-A1-2012/052948
- KATIA SNÉGAROFF ET AL: "Deprotonative Metalation of Chloro- and Bromopyridines Using Amido-Based Bimetallic Species and Regioselectivity-Computed CH Acidity Relationships", CHEMISTRY - A EUROPEAN JOURNAL, Bd. 17, Nr. 47, 18. November 2011 (2011-11-18), Seiten 13284-13297, XP055394620, ISSN: 0947-6539, DOI: 10.1002/chem.201101993
- DHAU JASPREET S ET AL: "A study on the BF3directed lithiation of 3-chloro- and 3-bromopyridine", TETRAHEDRON, Bd. 69, Nr. 48, 2013, Seiten 10284-10291, XP028761490, ISSN: 0040-4020, DOI: 10.1016/J.TET.2013.10.026

## Beschreibung

Die vorliegende Erfindung betrifft ein Verfahren zur Herstellung von halogenierten Pyridinderivaten der Formel (II) 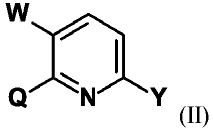 ausgehend von Verbindungen der Formel (I) 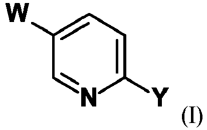 über Zwischenstufen der Formel (IIIa) oder (IIIb) 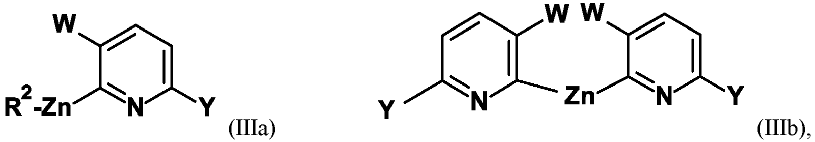 in denen die in den Formeln (I), (II), (IIIa) und (IIIb) angegebenen Strukturelemente die nachstehend angegebenen Bedeutungen haben. Ferner betrifft die Erfindung derartige halogenierte Pyridinderivate und Zwischenstufen.

Halogenierte Pyridinderivate der Formel (II) haben große technische Bedeutung für die pharmazeutische und die agrochemische Industrie und sind beispielsweise ein wichtiger Umsetzungspartner unter anderem bei der Herstellung von Verbindungen, die beispielsweise als Pestizide wirksam sind.

In der Literatur beschriebenen ist die Metallierung von Pyridinen in Gegenwart von Lithiumbeziehungsweise Magnesiumbasen (LDA, TMPLi, TMPMgCl·LiCl) und muss bei sehr tiefen Temperaturen (-78°C bis -100°C mit Lithium, -78°C bis -30°C mit Magnesium) durchgeführt werden, vgl. beispielsweise Tetrahedron 2001 (57), S. 4059ff, Journal of the American Chemical Society 2010 (132), S. 2410ff und Organic Letters 2011 (13), S. 2306ff. Ferner ist die Verwendung von der komplexen Manganbase TMP₂Mn·2MgCl₂·4LiCl für die Deprotonierung von 2,5-bis(substituierten) Pyridinen in Synlett 2015 (26), S. 1515ff beschrieben, allerdings nur, um oxidative Homokupplungen durchzuführen. Metallierungen in Gegenwart von Zinkbasen sind mit einigen substituierten Pyridinen an der 4. Position bekannt wie in Angewandte Chemie 2007 (46), S. 7685ff oder Organic Letters 2009 (11), S. 1837ff beschrieben, allerdings nicht mit 2,5-bis(substituierten) Pyridinen an der 6. Position.

Die bisher im Stand der Technik beschriebenen chemischen Syntheseverfahren von halogenierten Pyridinderivaten bedienen sich sehr häufig Methoden, die aus industrieller Sicht wirtschaftlich nicht zu realisieren sind und/oder andere Nachteile aufweisen.

Nachteilig sind insbesondere bei Lithium- und Magnesiumbasen die geringen chemischen Ausbeuten, die Durchführung bei sehr niedrigen Temperaturen sowie die schwierige Regio- und Chemo-Selektivität der Deprotoniereung aufgrund der hohen Reaktivität von diesen Reagenzien. Manchmal ist eine erforderliche Transmetallierung mit Zinksalzen wie zum Beispiel Zinkchlorid notwendig, um weitere selektive Reaktionen wie Negishi-Kreuzkupplungen durchzuführen, wie in Organic Letters 2008 (10), S. 2497ff beschrieben. Die Herstellung ist daher sehr teuer (viele Salze entstehen) und nicht für großtechnische kommerzielle Verfahren geeignet.

Im Hinblick auf die vorstehend geschilderten Nachteile besteht dringend Bedarf für ein vereinfachtes, technisch und ökonomisch durchführbares Verfahren zur Herstellung von halogenierten Pyridinderivaten, insbesondere von halogenierten Pyridinderivaten der Formel (II). Die mit diesem angestrebten Verfahren erhältlichen halogenierten Pyridinderivate sollen dabei vorzugsweise mit guter Ausbeute, hoher Reinheit und in ökonomischer Weise erhalten werden.

Überraschenderweise wurde gefunden, dass in einem Verfahren unter Verwendung einer Zink-metallorganischen Base halogenierte Pyridinderivate der Formel (II) vorteilhaft hergestellt werden können.

Gegenstand der vorliegenden Erfindung ist demnach ein Verfahren zur Herstellung von Verbindungen der Formel (II) 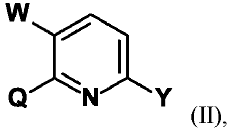 in welcher (Ausgestaltung 1)
Q und W unabhängig voneinander für Halogen stehen, und
Y für Halogen oder CO₂R¹ steht, wobei R¹ für (C₁-C₆)-Alkyl oder (C₁-C₆)-Haloalkyl steht,
dadurch gekennzeichnet, dass in einem ersten Verfahrensschritt a) eine Verbindung der Formel (I) 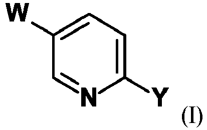
in welcher W und Y jeweils die oben genannten Bedeutungen haben,
mit einer Zink-metallorganischen Base der Struktur (NR³R⁴)-Zn-R² oder (NR³R⁴)₂-Zn, in welcher R² für Halogen steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 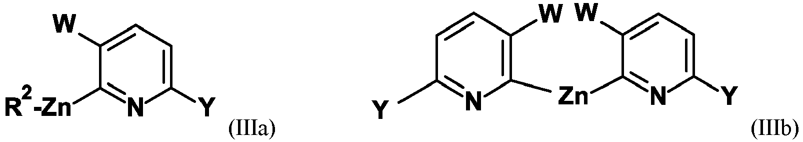
in welcher W, Y und R² jeweils die oben genannten Bedeutungen haben,
und diese Verbindung der Formel (IIIa) oder (IIIb) in einem zweiten Verfahrensschritt b) mit einer Verbindung der Struktur Q-X, in welcher X für Halogen steht und Q die oben genannte Bedeutung hat, zur Verbindung der Formel (II) umgesetzt wird, dadurch gekennzeichnet, dass Verfahrensschritt a) bei einer Temperatur zwischen 30 und 80 °C durchgeführt wird.

Bei der Verbindung Q-X handelt es sich, wie aus den Definitionen für Q und X hervorgeht, um eine Interhalogenverbindung, vorzugsweise um elementares Halogen.

Bevorzugte und besonders bevorzugte Bedeutungen der in den vorstehend erwähnten Formeln (I), (II) (IIIa) und (IIIb) des erfindungsgemäßen Verfahrens aufgeführten Reste Q, W, R¹, R², X, und Y werden im Folgenden erläutert, wobei die Zink-metallorganische Base weiter unten noch genau beschrieben wird, so dass die bevorzugten Ausgestaltungen der Base dort angegeben sind.
(Ausgestaltung 2)
   Q und X haben dieselbe Bedeutung und stehen bevorzugt jeweils für Iod oder Brom,
   W steht bevorzugt für Fluor oder Chlor,
   R² steht bevorzugt für Halogen, insbesondere Chlor, Brom oder Iod, und
   Y steht bevorzugt für Chlor, Brom oder CO₂R¹, wobei R¹ für (C₁-C₄)-Alkyl steht.
(Ausgestaltung 3)
   Q und X haben dieselbe Bedeutung und stehen besonders bevorzugt jeweils für Iod,
   W steht besonders bevorzugt für Fluor,
   R² steht besonders bevorzugt für Chlor, und
   Y steht besonders bevorzugt für Chlor, Brom oder CO₂R¹, wobei R¹ für Methyl steht.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt,

Vorteilhafterweise lassen sich die halogenierten Pyridinderivate der Formel (II) mit dem erfindungsgemäßen Verfahren mit guten Ausbeuten und in hoher Reinheit herstellen. Aufgrund der sehr guten funktionellen Gruppen-Toleranz von Zink-Reagenzien sind Zinkbasen sehr attraktiv. Regio- und chemoselektive Metallierungen von Pyridinen in Gegenwart von stoechiometrischen Mengen von selektiven Basen werden möglich, auch bei erhöhten Temperaturen, ohne dass eine Arin-Eliminierung stattfindet oder empfindliche funktionelle Gruppen angegriffen werden. Die als Zwischenstufe entstandene Zinkverbindung kann anschließend mit verschiedenen Elektrophilen abgefangen werden wie beispielsweise in Organic Letters 2009 (11), S. 1837ff beschrieben. Diese neu substituierten Pyridinderivate können dann als wertvolle Synthone weiter umgesetzt werden.

Insbesondere ist die sehr gute und unerwartete sehr selektive Regioselektivität der Metallierung vorteilhaft, die insbesondere bei höheren Temperaturen (beispielsweise bei Erhitzen auf ca. 40 bis 80°C, beispielsweise 60°C) an der 6. Position vom Pyridingerüst stattfindet. Das Erhitzen ist demnach insbesondere dann vorteilhaft, wenn die Regioselektivität weiter verbessert werden soll. Beispielsweise entsteht bei Raumtemperatur eine Mischung von mit Iod abgefangenen Pyridinen an den Positionen 6 und 4, wohingegen bei höherer Temperatur mit Iod abgefangene Pyridine an der Positionen 6 entstehen.

Das erfindungsgemäße Verfahren kann anhand des folgenden Schemas (I) erläutert werden:

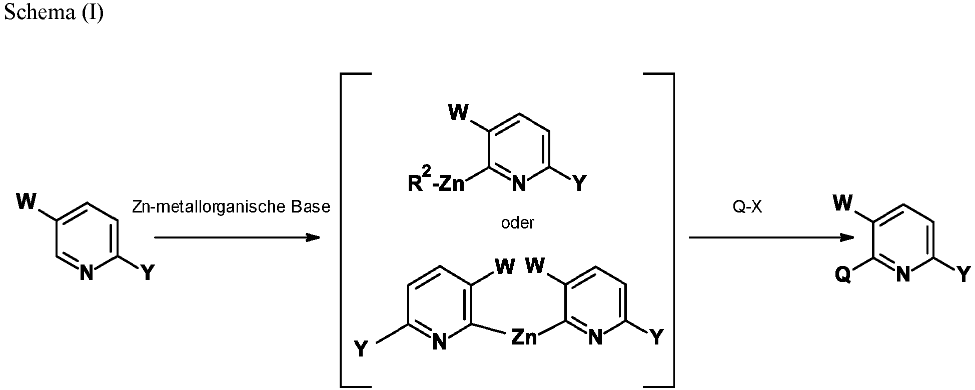

Hierin haben Q, W, R¹, R², X, und Y die vorstehend angegebenen Bedeutungen. Die in Klammern angegebenen Verbindungen stellen die Zwischenstufe (Formel IIIa beziehungsweise IIIb) dar, welche weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung der Formel (I) zur jeweiligen Zwischenstufe und Schritt b) die weitere Umsetzung der Zwischenstufe zur Verbindung der Formel (II) ist.

### Allgemeine Definitionen

Im Zusammenhang mit der vorliegenden Erfindung umfasst der Begriff Halogen (Hal), soweit nicht anders definiert, solche Elemente, die ausgewählt sind aus der Gruppe bestehend aus Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Der Begriff "Pivaloyl" beschreibt im Zusammenhang mit der vorliegenden Erfindung den deprotonierten Rest der Pivalinsäure (X) mit der Summenformel (CH₃)₃CCO₂H.

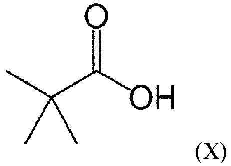

"O-Pivaloyl" bedeutet entsprechend, dass die Bindung des Pivaloylrestes über das deprotonierte Sauerstoffatom der Säuregruppe erfolgt.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-Hal) substituierte Alkyl-Gruppen sind beispielsweise ausgewählt aus Trifluormethyl (CF₃), Difluormethyl (CHF₂), CF3CH₂, ClCH₂ oder CF₃CCl₂.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Die Umsetzung der Verbindungen der Formel (I) zu Verbindungen der Formel (IIIa) oder Formel (IIIb) im ersten Verfahrensschritt (Schritt a)) erfolgt in Gegenwart einer Zink-metallorganischen Base der Struktur (NR³R⁴)-Zn-R² oder (NR³R⁴)₂-Zn, in welcher (Ausgestaltung B-1)
R² wie vorstehend (Ausgestaltung 1) definiert ist (daher für Halogen steht),
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl, Ethyl, n-Propyl und i-Propyl.
Bevorzugt ist, dass (Ausgestaltung B-2)
R² wie vorstehend als bevorzugt (Ausgestaltung 2) definiert ist (daher für Halogen steht, insbesondere für Chlor, Brom oder Iod),
R³ und R⁴ gemeinsam eine -(CH₂)₅-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl und Ethyl.
Besonders bevorzugt ist, dass (Ausgestaltung B-3)
R² wie vorstehend als besonders bevorzugt (Ausgestaltung 3) definiert ist (daher für Chlor steht) und
R³ und R⁴ gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR³R⁴) Tetramethylpiperidin (TMP) gemäß Formel (IV).

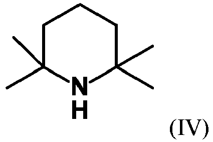

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (V) auf (Ausgestaltung B-4)

(V) (TMP)ₓZnCl₂₋ₓ ,

worin x für die Zahl 1 oder 2 steht. Hierunter wiederum bevorzugt sind Basen mit x=1 (Ausgestaltung B-5) gemäß Formel (VI):

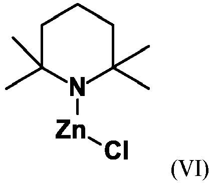

In einer weiteren bevorzugten Ausführungsform des erfindungsgemäßen Verfahrens liegt die metallorganische Base in Verbindung mit Alkali- oder Erdalkalihalogeniden vor. Dies gilt insbesondere für Basen der Formeln (V) und (VI). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte metallorganische Basen sind demnach TMP ZnCl·LiCl oder (TMP)₂ Zn·2LiCl (Ausgestaltung B-6). Am meisten bevorzugt ist TMP ZnCl·LiCl (VII; Ausgestaltung B-7).

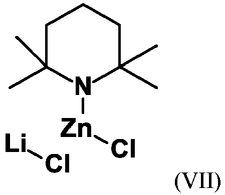

Beispielhaft werden in nachfolgender Tabelle 1 konkrete Kombinationen von Verbindungen der Formeln (I), (II) und (IIIa) bzw. (IIIb) mit erfindungsgemäßen Basen genannt, die in einem erfindungsgemäßen Verfahren zur Anwendung kommen können. Da in einigen Ausgestaltungen das Strukturelement R² sowohl in der erfindungsgemäßen Base als auch in der Verbindung der Formel (IIIa) vorliegt, gilt für R² jeweils die engste Definition.

**Tabelle 1:**

| Nummer | Verbindungen der Formeln (I), (II) und (IIIa) bzw. (IIIb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |

Vorzugsweise wird die metallorganische Base in dem erfindungsgemäßen Verfahren in einer Gesamtmenge von 0,5 bis 5 Äquivalenten, vorzugsweise von 0,8 bis 2 Äquivalenten, weiter bevorzugt von 1 bis 1,5 Äquivalenten und besonders bevorzugt von 1,0 bis 1,2 Äquivalenten, bezogen auf die Verbindung der Formel (I), eingesetzt. Ein Vorteil des erfindungsgemäßen Verfahrens ist es diesbezüglich, dass die metallorganische Base in nahezu stöchiometrischen Mengen eingesetzt werden kann.

In Abängigkeit davon, ob in der eingesetzten Zink-metallorganischen Base das Strukturelement (NR³R⁴) einfach oder zweifach vorhanden ist, entstehen Zwischenverbindungen der Formel (IIIa) beziehungsweise der Formel (IIIb) in Verfahrensschritt a).

Die Umsetzung der Verbindungen der Formel (IIIa) beziehungsweise (IIIb) zu Verbindungen der Formel (II) im zweiten Verfahrensschritt (Schritt b)) erfolgt in Gegenwart einer Verbindung Q-X, in welcher Q und X jeweils die vorstehend genannten Bedeutungen haben. Da sowohl Q als auch X für Halogen stehen, handelt es sich um eine Interhalogenverbindung. Q und X müssen nicht notwendigerweise für das gleiche Halogen stehen. Beispielsweise kann Q für Iod oder Brom stehen und X für Chlor, Brom oder Iod. Vorzugsweise handelt es sich bei der Verbindung Q-X aber um ein elementares Halogen, insbesondere F₂, Cl₂, Br₂ oder I₂. Besonders bevorzugt sind I₂ oder Br₂, ganz besonders bevorzugt ist I₂.

Vorzugsweise wird die Verbindung Q-X in dem erfindungsgemäßen Verfahren in einer Gesamtmenge von 0,5 bis 10,0 Äquivalenten, vorzugsweise von 0,8 bis 5 Äquivalenten, weiter bevorzugt von 1 bis 2,5 Äquivalenten und besonders bevorzugt von 1,0 bis 1,5 Äquivalenten, bezogen auf die Verbindung der Formel (I), eingesetzt.

Die erfindungsgemäße Umsetzung der Verbindungen der Formel (I) zu Verbindungen der Formel (IIIa) beziehungsweise (IIIb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrilidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Das Lösungsmittel kann ferner entgast (sauerstoff-frei) sein.

Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.

Die Umsetzung in Verfahrensschritt a) wird bei einer Temperatur zwischen 30°C und 80°C und zunehmend bevorzugt zwischen 30°C und 70°C, zwischen 40°C und 68°C und ganz besonders bevorzugt zwischen 50°C und 65°C, beispielsweise bei 60°C, durchgeführt.

Die Umsetzung in Verfahrensschritt b) wird im Allgemeinen bei einer Temperatur zwischen 0°C und 80°C und zunehmend bevorzugt zwischen 10°C und 70°C, zwischen 15°C und 60°C, zwischen 20°C und 50°C, zwischen 20°C und 40°C und ganz besonders bevorzugt zwischen 20°C und 35°C, beispielsweise bei Raumtemperatur bzw. 25°C, durchgeführt.

Die Reaktion wird üblicherweise bei Normaldruck durchgeführt, kann aber auch bei erhöhtem bzw. vermindertem Druck durchgeführt werden.

Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Eine besonders bevorzugte Ausführungsform des erfindungsgemäßen Verfahrens kann anhand des folgenden Schemas (II) erläutert werden:

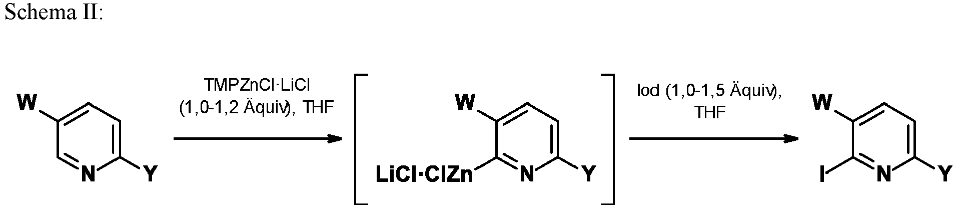

Hierin haben W und Y die vorstehend angegebenen Bedeutungen. Die in Klammern angegebene Verbindung stellt die entsprechende Zwischenstufe der Formel IIIa dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. Iod (I₂).

Ein weiterer Gegenstand der vorliegenden Erfindung sind Verbindungen der Formel (IIIa) 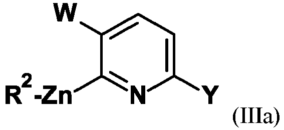 in welcher
W für Fluor steht,
Y für Halogen oder CO₂R¹ steht, wobei R¹ für (C₁-C₆)-Alkyl oder (C₁-C₆)-Haloalkyl steht, und
R² für Halogen steht.
Bevorzugte und besonders bevorzugte Bedeutungen der in der vorstehend erwähnten Formel (IIIa) aufgeführten Reste werden im Folgenden erläutert.
W steht bevorzugt für Fluor,
Y steht bevorzugt für Chlor, Brom oder CO₂R¹, wobei R¹ für (C₁-C₄)-Alkyl steht, und
R² steht bevorzugt für Halogen, insbesondere Chlor, Brom und Iod.
W steht besonders bevorzugt für Fluor,
Y steht besonders bevorzugt für Chlor, Brom oder CO₂R¹, wobei R¹ für Methyl steht, und
R² steht besonders bevorzugt für Chlor.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Unter den Verbindungen der Formel (IIIa) sind die folgenden Verbindungen ganz besonders bevorzugt, wobei die jeweilige Verbindung alleine oder als Lithiumchloridkomplex vorliegen kann:

| | | |
|---|---|---|
| IIIa-1: | 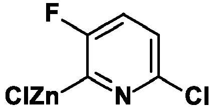 | 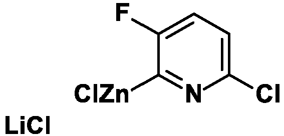 |
| | Chlor(6-chlor-3-fluorpyridin-2-yl)zink | Chlor(6-chlor-3-fluorpyridin-2-yl)zink Lithiumchlorid Komplex |
| IIIa-2: | 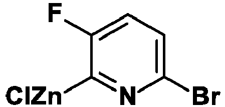 | 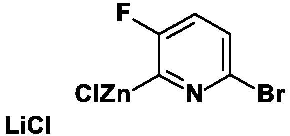 |
| | 6-Brom-3-fluorpyridin-2-yl)(chlor)zink | 6-Brom-3-fluorpyridin-2-yl)(chlor)zink Lithiumchlorid Komplex) |
| IIIa-3: | 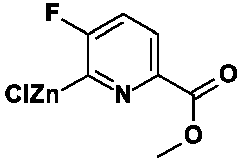 | 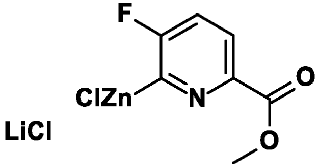 |
| | Chlor[3-fluor-6-(methoxycarbonyl)pyridin-2-yl]zink | Chlor[3-fluor-6-(methoxycarbonyl)pyridin-2-yl]zink Lithiumchlorid Komplex |

Ein weiterer Gegenstand der vorliegenden Erfindung sind Verbindungen der Formel (IIIb) 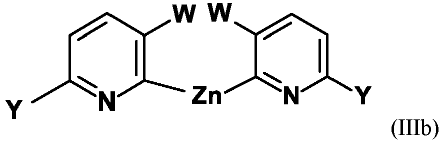 in welcher
W für Fluor steht, und
Y für Halogen oder CO₂R¹ steht, wobei R¹ für (C₁-C₆)-Alkyl oder (C₁-C₆)-Haloalkyl steht.
Bevorzugte und besonders bevorzugte Bedeutungen der in der vorstehend erwähnten Formel (IIIb) aufgeführten Reste werden im Folgenden erläutert.
W steht bevorzugt für Fluor, und
Y steht bevorzugt für Chlor, Brom oder CO₂R¹, wobei R¹ für (C₁-C₄)-Alkyl steht.
W steht besonders bevorzugt für Fluor, und
Y steht besonders bevorzugt für Chlor, Brom oder CO₂R¹, wobei R¹ für Methyl steht.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Ein weiterer Gegenstand der vorliegenden Erfindung sind Verbindungen der Formel (II) 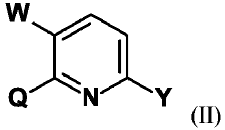 in welcher
Q für Iod steht,
W für Fluor steht, und
Y für Halogen oder CO₂R¹ steht, wobei R¹ für (C₁-C₆)-Alkyl oder (C₁-C₆)-Haloalkyl steht.
Bevorzugte und besonders bevorzugte Bedeutungen der in der vorstehend erwähnten Formel (II) aufgeführten Reste werden im Folgenden erläutert.
Y steht bevorzugt für Chlor, Brom oder CO₂R¹, wobei R¹ für (C₁-C₄)-Alkyl steht.
Y steht besonders bevorzugt für Chlor, Brom oder CO₂R¹, wobei R¹ für Methyl steht.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Unter den Verbindungen der Formel (II) sind die folgenden Verbindungen ganz besonders bevorzugt:

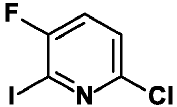

### (II-1, 6-Chlor-3-fluor-2-iodpyridin)

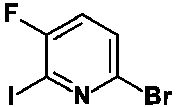

### (II-2, 6-Brom-3-fluor-2-iodpyridin)

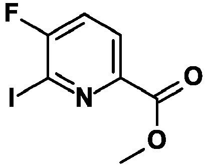

### (II-3, Methyl-5-fluor-6-iodpyridin-2-carboxylat)

Aus diesen Verbindungen der Formeln (II-1) bis (II-3) ergeben sich die zugehörigen Edukte (1-1 bis 1-3) der Formel (I) des erfindungsgemäßen Verfahrens, welche jeweils ganz besonders bevorzugte Verbindungen der Formel (I) sind.

Die vorliegende Erfindung wird anhand der nachfolgenden Beispiele näher erläutert, wobei die Beispiele nicht in einer die Erfindung einschränkenden Weise zu interpretieren sind.

### Beispiel 1

### Synthese von 6-Chlor-3-fluor-2-iodpyridin:

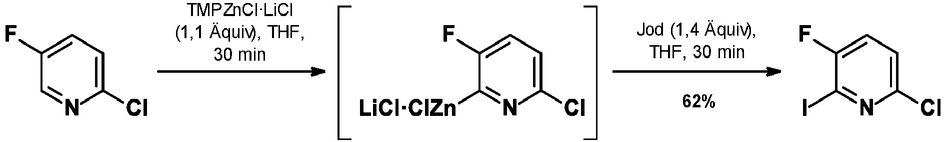

2-Chlor-5-fluorpyridin (132 mg, 1.0 mmol), gelöst in THF (2 ml), wird mit TMPZnCl·LiCl (1.31 M in THF, 0.84 ml, 1.1 mmol) bei 60 °C unter Argon versetzt; diese Reaktionslösung wird 30 min gerührt. Anschließend wird Iod (355 mg in 4 ml THF) bei 25 °C zugegeben und die Lösung während 30 min weitergerührt. Nach üblicher Aufarbeitung durch Zugabe von gesättigter Ammoniumchlorid- und Natriumthiosulfat-Lösungen wird das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 6-Chlor-3-fluor-2-iodpyridin (158 mg, 62%) als gelben Feststoff. HPLC-MS: logP = 2,53; Masse (m/z): keine Ionisierung; 1HNMR (D6-DMSO): δ 7,80 (m, 1H), 7,61 (m, 1H).

### Beispiel 2

### Synthese von Methyl-5-fluor-6-iodpyridin-2-carboxylat:

Methyl-5-fluorpyridin-2-carboxylat (155 mg, 1.0 mmol), gelöst in THF (2 ml), wird mit TMPZnCl·LiCl (1.31 M in THF, 0.84 ml, 1.1 mmol) bei 60 °C unter Argon versetzt; diese Reaktionslösung wird 30 min gerührt. Anschließend wird Iod (355 mg in 4 ml THF) bei 25 °C zugegeben und die Lösung während 30 min weitergerührt. Nach üblicher Aufarbeitung und säulenchromatographischer Reinigung (Ethylacetat/Cyclohexan) erhält man Methyl-5-fluor-6-iodpyridin-2-carboxylat (197 mg, 70%) als gelben Feststoff. HPLC-MS: logP = 1,85; Masse (m/z): 281,9; 1HNMR (D6-DMSO): δ 8,10 (m, 1H), 7,86 (m, 1H), 3,89 (s, 3H).

### Beispiel 3

### Synthese von 6-Brom-3-fluor-2-iodpyridin:

2-Brom-5-fluorpyridin (880 mg, 5.0 mmol), gelöst in THF (5 ml), wird mit TMPZnCl·LiCl (1.31 M in THF, 4.2 ml, 5.5 mmol) bei 60 °C unter Argon versetzt; diese Reaktionslösung wird 30 min gerührt. Anschließend wird Iod (1,78 g in 4 ml THF) bei 25 °C zugegeben und die Lösung während 30 min weitergerührt. Nach üblicher Aufarbeitung durch Zugabe von gesättigter Ammoniumchlorid- und Natriumthiosulfat-Lösungen wird das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhält man 6-Brom-3-fluor-2-iodpyridin (1,31 g, 87%) als gelben Feststoff. HPLC-MS: logP = 2,67; Masse (m/z): keine Ionisierung; 1HNMR (D6-DMSO): δ 7,72 (m, 2H).

## Patentansprüche

1. Verfahren zur Herstellung von Verbindungen der Formel (II) 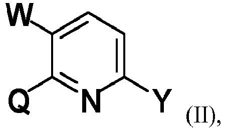 in welcher
Q und W unabhängig voneinander für Halogen stehen, und
Y für Halogen oder CO₂R¹ steht, wobei R¹ für (C₁-C₆)-Alkyl oder (C₁-C₆)-Haloalkyl steht,
**dadurch gekennzeichnet, dass** in einem ersten Verfahrensschritt a) eine Verbindung der Formel (I) 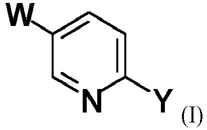
in welcher W und Y jeweils die oben genannten Bedeutungen haben,
mit einer Zink-metallorganischen Base der Struktur (NR³R⁴)-Zn-R² oder (NR³R⁴)₂-Zn, in welcher
R² für Halogen steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 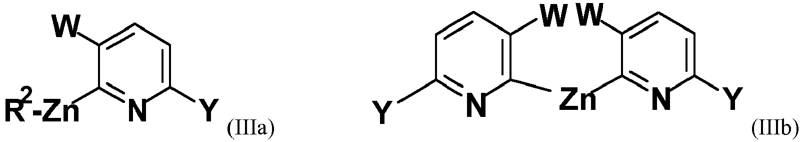
in welcher W, Y und R² jeweils die oben genannten Bedeutungen haben,
und diese Verbindung der Formel (IIIa) oder (IIIb) in einem zweiten Verfahrensschritt b) mit einer Verbindung der Struktur Q-X, in welcher X für Halogen steht und Q die oben genannte Bedeutung hat, zur Verbindung der Formel (II) umgesetzt wird,
**dadurch gekennzeichnet, dass** Verfahrensschritt a) bei einer Temperatur zwischen 30°C und 80°C durchgeführt wird.

2. Verfahren gemäß Anspruch 1, **dadurch gekennzeichnet, dass**
Q und X dieselbe Bedeutung haben und jeweils für Iod oder Brom stehen,
W für Fluor oder Chlor steht,
R² für Halogen, insbesondere Chlor, Brom oder Iod, steht, und
Y für Chlor, Brom oder CO₂R¹ steht, wobei R¹ für (C₁-C₄)-Alkyl steht.

3. Verfahren gemäß einem der Ansprüche 1 oder 2, **dadurch gekennzeichnet, dass**
R³ und R⁴ gemeinsam eine -(CH₂)₅-Gruppe bilden, wobei jede dieser Gruppen optional durch 1, 2, 3 oder 4 R⁵-Radikale substituiert sein kann, und
R⁵ ausgewählt ist aus der Gruppe bestehend aus Methyl und Ethyl.

4. Verfahren gemäß einem der Ansprüche 1 bis 3, **dadurch gekennzeichnet, dass**
Q und X dieselbe Bedeutung haben und jeweils für Iod stehen,
W für Fluor steht,
R² für Chlor steht, und
Y für Chlor, Brom oder CO₂R¹ steht, wobei R¹ für Methyl steht.

5. Verfahren gemäß einem der Ansprüche 1 bis 4, **dadurch gekennzeichnet, dass**
R³ und R⁴ gemeinsam eine -(CH₂)₅-Gruppe bilden, die durch 4 Methylgruppen substituiert ist.

6. Verfahren gemäß einem der Ansprüche 1 bis 5, **dadurch gekennzeichnet, dass** es sich bei der Zink-metallorganischen Base um eine Verbindung der Formel (V) handelt
(V) (TMP)ₓZnCl₂₋ₓ ,
worin x für die Zahl 1 oder 2 steht.

7. Verfahren gemäß einem der Ansprüche 1 bis 6, **dadurch gekennzeichnet, dass** die Zink-metallorganische Base in Verbindung mit einem Alkali- oder Erdalkalihalogenid, vorzugsweise Lithiumchlorid oder Magnesiumchlorid, vorliegt.

8. Verfahren gemäß einem der Ansprüche 1 bis 7, **dadurch gekennzeichnet, dass** die Zink-metallorganische Base in einer Gesamtmenge von 0,5 bis 5 Äquivalenten, bezogen auf die Verbindung der Formel (I), eingesetzt wird.

9. Verfahren gemäß einem der Ansprüche 1 bis 8, **dadurch gekennzeichnet, dass** es sich bei der Verbindung Q-X um ein elementares Halogen, insbesondere um F₂, Cl₂, Br₂ oder I₂, handelt.

10. Verfahren gemäß einem der Ansprüche 1 bis 9, **dadurch gekennzeichnet, dass** die Verbindung Q-X in einer Gesamtmenge von 0,5 bis 10,0 Äquivalenten, bezogen auf die Verbindung der Formel (I), eingesetzt wird.

11. Verfahren gemäß einem der Ansprüche 1 bis 10, **dadurch gekennzeichnet, dass** es in Gegenwart eines Lösungsmittels durchgeführt wird, welches ausgewählt ist aus der Gruppe bestehend aus Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoff, aromatischer Kohlenwasserstoff, Chlorkohlenwasserstoff, Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, 1,2-Dichlorbenzol, Chlortoluol, Trichlorbenzol; 4-Methoxybenzol, fluoriertes Aliphat, fluorierter Aromat, Trichlortrifluorethan, Benzotrifluorid und 4-Chlorbenzotrifluorid, oder einer Mischung aus mindestens zwei dieser Lösungsmittel untereinander.

12. Verfahren gemäß Anspruch 11, **dadurch gekennzeichnet, dass** das Lösungsmittel THF oder N,N-Dimethylformamid (DMF) ist.

13. Verfahren gemäß einem der Ansprüche 1 bis 12, **dadurch gekennzeichnet, dass** Verfahrensschritt b) bei einer Temperatur zwischen 0°C und 80°C durchgeführt wird.

14. Verbindung der Formel (IIIa) 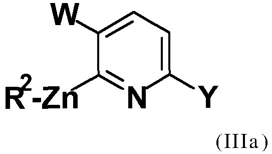 in welcher
W für Fluor steht,
Y für Halogen oder C(O)₂R¹ steht, wobei R¹ für (C₁-C₆)-Alkyl oder (C₁-C₆)-Haloalkyl steht, und
R² für Halogen steht.

15. Verbindung der Formel (IIIb) 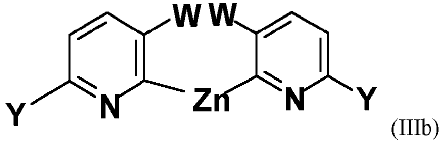 in welcher
W für Fluor steht, und
Y für Halogen oder CO₂R¹ steht, wobei R¹ für (C₁-C₆)-Alkyl oder (C₁-C₆)-Haloalkyl steht.

16. Verbindung der Formel (II) 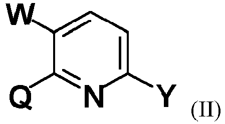 in welcher
Q für Iod steht,
W für Fluor steht, und
Y für Halogen oder C(O)₂R¹ steht, wobei R¹ für (C₁-C₆)-Alkyl oder (C₁-C₆)-Haloalkyl steht.

## Claims

1. Process for preparing compounds of the formula (II) 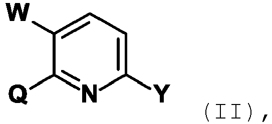 in which
Q and W are each independently halogen, and
Y is halogen or CO₂R¹, where R¹ is (C₁-C₆)-alkyl or (C₁-C₆)-haloalkyl,
**characterized in that**, in a first process step a), a compound of the formula (I) 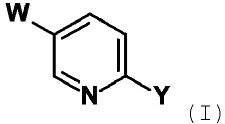
in which W and Y each have the definitions given above, is reacted with an organozinc base of the structure (NR³R⁴)-Zn-R² or (NR³R⁴)₂-Zn in which
R² is halogen and
R³ and R⁴ together form a -(CH₂)₄-, -(CH₂)₅- or - (CH₂)₂O(CH₂)₂- group, where each of these groups may optionally be substituted by 1, 2, 3 or 4 R⁵ radicals and R⁵ is selected from the group consisting of methyl, ethyl, n-propyl and i-propyl,
to give a compound of the formula (IIIa) or the formula (IIIb) 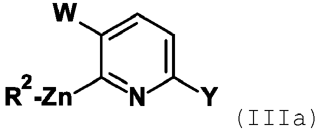 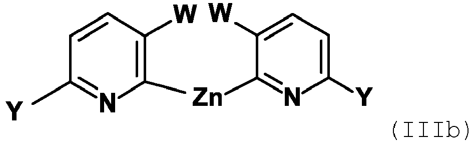
in which W, Y and R² each have the definitions given above,
and this compound of the formula (IIIa) or (IIIb) is reacted in a second process step b) with a compound of the structure Q-X in which X is halogen and Q has the abovementioned definition to give the compound of the formula (II),
**characterized in that** process step a) is conducted at a temperature between 30°C and 80°C.

2. Process according to Claim 1, **characterized in that**
Q and X have the same definition and are each iodine or bromine,
W is fluorine or chlorine,
R² is halogen, especially chlorine, bromine or iodine, and
Y is chlorine, bromine or CO₂R¹, where R¹ is (C₁-C₄)-alkyl.

3. Process according to either of Claims 1 and 2, **characterized in that**
R³ and R⁴ together form a -(CH₂)₅- group, where each of these groups may optionally be substituted by 1, 2, 3 or 4 R⁵ radicals, and
R⁵ is selected from the group consisting of methyl and ethyl.

4. Process according to any of Claims 1 to 3, **characterized in that**
Q and X have the same definition and are each iodine, W is fluorine,
R² is chlorine, and
Y is chlorine, bromine or CO₂R¹, where R¹ is methyl.

5. Process according to any of Claims 1 to 4, **characterized in that**
R³ and R⁴ together form a -(CH₂)₅- group substituted by 4 methyl groups.

6. Process according to any of Claims 1 to 5, **characterized in that** the organozinc base is a compound of the formula (V)
(V) (TMP)ₓ ZnCl₂₋ₓ ,
in which x is the number 1 or 2.

7. Process according to any of Claims 1 to 6, **characterized in that** the organozinc base is present in conjunction with an alkali metal halide or alkaline earth metal halide, preferably lithium chloride or magnesium chloride.

8. Process according to any of Claims 1 to 7, **characterized in that** the organozinc base is used in a total amount of 0.5 to 5 equivalents, based on the compound of the formula (I).

9. Process according to any of Claims 1 to 8, **characterized in that** the compound Q-X is an elemental halogen, especially F₂, Cl₂, Br₂ or I₂.

10. Process according to any of Claims 1 to 9, **characterized in that** the compound Q-X is used in a total amount of 0.5 to 10.0 equivalents, based on the compound of the formula (I).

11. Process according to any of Claims 1 to 10, **characterized in that** it is conducted in the presence of a solvent selected from the group consisting of tetrahydrofuran (THF), 1,4-dioxane, diethyl ether, diglyme, methyl tert-butyl ether (MTBE), tert-amyl methyl ether (TAME), 2-methyl-THF, toluene, xylenes, mesitylene, ethylene carbonate, propylene carbonate, N,N-dimethylacetamide, N,N-dimethylformamide (DMF), N-methylpyrrolidone (NMP), N-ethyl-2-pyrrolidone (NEP), N-butyl-2-pyrrolidone (NBP); N,N'-dimethylpropyleneurea (DMPU), halohydrocarbon, aromatic hydrocarbon, chlorohydrocarbon, tetrachloroethylene, tetrachloroethane, dichloropropane, methylene chloride, dichlorobutane, chloroform, carbon tetrachloride, trichloroethane, trichloroethylene, pentachloroethane, difluorobenzene, 1,2-dichloroethane, chlorobenzene, bromobenzene, dichlorobenzene, 1,2-dichlorobenzene, chlorotoluene, trichlorobenzene; 4-methoxybenzene, fluorinated aliphatic, fluorinated aromatic, trichlorotrifluoroethane, benzotrifluoride and 4-chlorobenzotrifluoride, or a mixture of at least two of these solvents with one another.

12. Process according to Claim 11, **characterized in that** the solvent is THF or N,N-dimethylformamide (DMF).

13. Process according to any of Claims 1 to 12, **characterized in that** process step b) is conducted at a temperature between 0°C and 80°C.

14. Compound of the formula (IIIa) 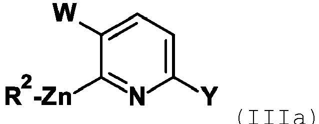 in which
W is fluorine,
Y is halogen or C(O)₂R¹, where R¹ is (C₁-C₆)-alkyl or (C₁-C₆)-haloalkyl, and
R² is halogen.

15. Compound of the formula (IIIb) 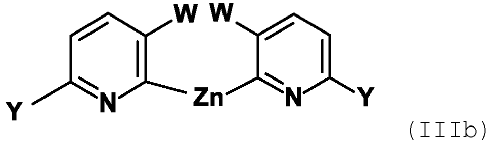 in which
W is fluorine, and
Y is halogen or CO₂R¹, where R¹ is (C₁-C₆)-alkyl or (C₁-C₆)-haloalkyl.

16. Compound of the formula (II) 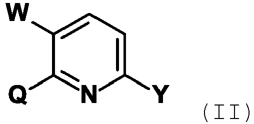 in which
Q is iodine,
W is fluorine, and
Y is halogen or C(O)₂R¹, where R¹ is (C₁-C₆)-alkyl or (C₁-C₆)-haloalkyl.

## Revendications

1. Procédé de préparation de composés de formule (II) 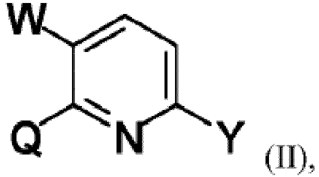 dans laquelle
Q et W représentent chacun indépendamment de l'autre un halogène, et
Y représente un halogène ou CO₂R¹, R¹ représentant un alkyle en C₁-C₆ ou un halogénoalkyle en C₁-C₆,
**caractérisé en ce que**, dans une première étape a), on fait réagir un composé de formule (I) 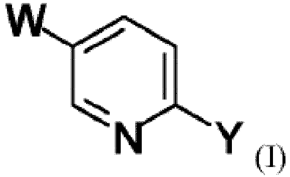
dans laquelle W et Y ont chacun les significations données ci-dessus,
avec une base organozincique de structure (NR³R⁴)-Zn-R² ou (NR³R⁴)₂-Zn, dans laquelle
R² représente un halogène et
R³ et R⁴ forment ensemble un groupe -(CH₂)₄-, -(CH₂)₅- ou - (CH₂)₂O(CH₂)₂-, chacun de ces groupes pouvant éventuellement être substitué par 1, 2, 3 ou 4 radicaux R⁵, et R⁵ étant choisi dans le groupe consistant en un méthyle, un éthyle, un n-propyle et un isopropyle,
pour obtenir un composé de formule (IIIa) ou de formule (IIIb), 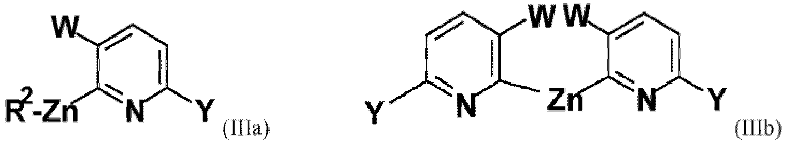
dans laquelle W, Y et R² ont chacun les significations données ci-dessus,
et on fait réagir ce composé de formule (IIIa) ou (IIIb), dans une deuxième étape b), avec un composé de structure Q-X dans laquelle X représente un halogène et Q a les significations données ci-dessus, pour donner le composé de formule (II),
**caractérisé en ce que** l'étape a) est mise en œuvre à une température entre 30 °C et 80 °C.

2. Procédé selon la revendication 1, **caractérisé en ce que**
Q et X ont la même signification et représentent chacun un iode ou un brome,
W représente un fluor ou un chlore,
R² représente un halogène, en particulier un chlore, un brome ou un iode, et
Y représente un chlore, un brome ou CO₂R¹, R¹ représentant un alkyle en C₁-C₄.

3. Procédé selon l'une des revendications 1 ou 2, **caractérisé en ce que** R³ et R⁴ forment ensemble un groupe -(CH₂)₅-, chacun de ces groupes pouvant éventuellement être substitué par 1, 2, 3 ou 4 radicaux R⁵, et
R⁵ est choisi dans le groupe consistant en un méthyle et un éthyle.

4. Procédé selon l'une des revendications 1 à 3, **caractérisé en ce que**
Q et X ont la même signification et représentent chacun un iode,
W représente un fluor,
R² représente un chlore, et
Y représente un chlore, un brome ou CO₂R¹, R¹ représentant un méthyle.

5. Procédé selon l'une des revendications 1 à 4, **caractérisé en ce que** R³ et R⁴ forment ensemble un groupe -(CH₂)₅, qui est substitué par 4 groupes méthyle.

6. Procédé selon l'une des revendications 1 à 5, **caractérisé en ce que** la base organozincique est un composé de formule (V)
(V) (TMP)ₓ ZnCl₂₋ₓ,
dans laquelle x représente le nombre 1 ou 2.

7. Procédé selon l'une des revendications 1 à 6, **caractérisé en ce que** la base organozincique se présente en liaison avec un halogénure d'un métal alcalin ou alcalino-terreux, de préférence avec du chlorure de lithium ou du chlorure de magnésium.

8. Procédé selon l'une des revendications 1 à 7, **caractérisé en ce que** la base organozincique est utilisée en une quantité totale de 0,5 à 5 équivalents, par rapport au composé de formule (I).

9. Procédé selon l'une des revendications 1 à 8, **caractérisé en ce que** le composé Q-X est un halogène élémentaire, en particulier F₂, Cl₂, Br₂ ou I₂.

10. Procédé selon l'une des revendications 1 à 9, **caractérisé en ce que** le composé Q-X est utilisé en une quantité totale de 0,5 à 10,0 équivalents, par rapport au composé de formule (I).

11. Procédé selon l'une des revendications 1 à 10, **caractérisé en ce qu'**il est mis en œuvre en présence d'un solvant qui est choisi dans le groupe consistant en les solvants suivants : tétrahydrofurane (THF), 1,4-dioxane, diéthyléther, diglyme, méthyl-tertbutyléther (MTBE), tert-amyl-méthyléther (TAME), 2-méthyl-THF, toluène, xylènes, mésitylène, carbonate d'éthylène, carbonate de propylène, N,N-diméthylacétamide, N,N-diméthylformamide (DMF), N-méthylpyrrolidone (NMP), N-éthyl-2-pyrrolidone (NEP), N-butyl-2-pyrrolidone (NBP) ; N,N'-diméthylpropylène-urée (DMPU), halogénohydrocarbure, hydrocarbure aromatique, hydrocarbure chloré, tétrachloréthylène, tétrachloréthane, dichloropropane, chlorure de méthylène, dichlorobutane, chloroforme, tétrachlorure de carbone, trichloréthane, trichloréthylène, pentachloréthane, difluorobenzène, 1,2-dichloroéthane, chlorobenzène, bromobenzène, dichlorobenzène, 1,2-dichlorobenzène, chlorotoluène, trichlorotoluène ; 4-méthoxybenzène, aliphatique fluoré, aromatique fluoré, trichlorotrifluoréthane, benzotrifluorure et 4-chlorobenzotrifluorure, ou un mélange d'au moins deux de ces solvants l'un avec l'autre.

12. Procédé selon la revendication 11, **caractérisé en ce que** le solvant est le THF ou le N,N-diméthylformamide (DMF).

13. Procédé selon l'une des revendications 1 à 12, **caractérisé en ce que** l'étape b) est mise en œuvre à une température entre 0 °C et 80 °C.

14. Composé de formule (IIIa) 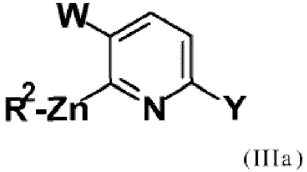 dans laquelle
W représente un fluor,
Y représente un halogène ou C(O)₂R¹, R¹ représentant un alkyle en C₁-C₆ ou un halogénoalkyle en C₁-C₆, et
R² représentant un halogène.

15. Composé de formule (IIIb) 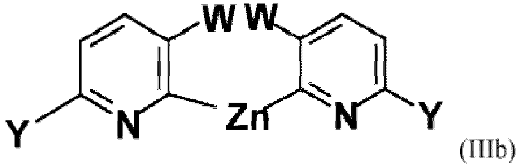 dans laquelle
W représente un fluor, et
Y représente un halogène ou CO₂R¹, R¹ représentant un alkyle en C₁-C₆ ou un halogénoalkyle en C₁-C₆.

16. Composé de formule (II) 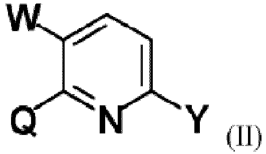 dans laquelle
Q représente un iode,
W représente un fluor, et
Y représente un halogène ou C(O)₂R¹, R¹ représentant un alkyle en C₁-C₆ ou un halogénoalkyle en C₁-C₆.
